# EUROPEAN PATENT APPLICATION

(11) **EP 4 201 406 A1**
(43) Date of publication of application: **28.06.2023**
(21) Application number: 21858508.1
(22) Date of filing: 06.08.2021
(51) Int. Cl.: A61K 31/506, A61P 25/28, A23L 33/10

(54) **PHARMACEUTICAL COMPOSITION FOR PREVENTION OR TREATMENT OF DEGENERATIVE BRAIN DISEASES COMPRISING ABEMACICLIB AS ACTIVE INGREDIENT**

(30) Priority: 18.08.2020 KR 20200103272; 05.08.2021 KR 20210103197
(71) Applicant: Daegu Gyeongbuk Institute Of Science and Technology, Daegu 42988 (KR)
(72) Inventor: HOE, Hyang Sook, Daegu 41064 (KR); LEE, Hyun Ju, Dalseong-gun Daegu 42960 (KR)
(74) Representative: Bittner, Thomas L.
(86) International application number: PCT/KR2021/010414
(87) International publication number: WO 2022/039421

(57) **Abstract**

The present invention relates to a pharmaceutical composition for the prevention or treatment of degenerative brain diseases, comprising abemaciclib as an active ingredient and, in particular, to: a pharmaceutical composition for the prevention or treatment of degenerative brain diseases, comprising abemaciclib or a salt thereof as an active ingredient; and a health functional food for the prevention or amelioration of degenerative brain diseases. Abemaciclib according to the present invention has excellent activity of inhibiting an inflammatory cytokine, i.e., COX-2, IL-1β, IL-6, or iNOS in brain neurons, has excellent activity of inhibiting TLR4 signaling related to inflammatory responses and inhibiting the level of p-STAT3, can inhibit the activity of microglia or astrocytes by LPS, also has the activities of inhibiting amyloid plaques, increasing the number of dendrites, and inhibiting the phosphorylation of tau protein, and can improve the long-term memory of mice with induced degenerative brain diseases. Therefore, abemaciclib of the present invention is effective in use as an active ingredient of medicines and health functional foods for preventing, ameliorating, or treating degenerative brain diseases or neuroinflammatory diseases.

## Description

### Technical Field

The present invention relates to a pharmaceutical composition for preventing or treating a degenerative brain disease containing abemaciclib as an active ingredient.

### Background

Degenerative brain disease refers to a neurodegenerative disease that occurs in the brain with age and may be classified depending on the main symptoms and the affected brain region. Representative degenerative brain diseases include Alzheimer's disease, Parkinson's disease and the like. Degenerative brain diseases are known to be caused by neurodegeneration due to aging and the death of neurons due to protein aggregation due to genetic and environmental factors.

In addition, degenerative brain diseases are characterized in that the death or degeneration of specific brain cells progresses temporarily or over a long period of time. It is known that dead brain cells do not regenerate, thus eventually leading to fatal loss of brain function. In particular, brain dysfunction accompanied by progressive deterioration of cognitive, sensory, motor, and systemic functions eventually causes changes in personality and behaviors, thus making patients unable to take care of themselves. Oxidative toxicity, excitatory toxicity, and apoptosis due to oxidative stress have been proposed as the main pathways of brain cell death, and each thereof induces cell death through a specific signaling pathway. Specifically, oxidative damage to proteins, nucleic acids, and lipids after accumulation of reactive oxygen species has been suggested as the main cause of brain cell death in patients with stroke, brain injury, Alzheimer's disease (AD), and Parkinson's disease. In particular, oxidative stress caused by free radicals has been reported as the main cause of cell death in each tissue in the body and has also been suggested as one of the main mechanisms of cell death in brain nerve diseases (Schapira, A.H., Curr. Opin. Neurol., 9(4):260-264, 1996).

In addition, it has been reported that microglia and astrocyte activation is related to the onset and progression of degenerative cranial nerve diseases, and microglia are known to be immune cells in the central nervous system (CNS) and be activated by external stimuli to induce immune and inflammatory responses. Microglia are cells that perform the primary immune function in the central nervous system. Microglia retain the shapes of thin and long spines and thin cell bodies, whereas when foreign toxins naturally-derived internal toxins are present, they are converted to activated forms having thick and short branches and obese cell bodies in order to protect nerve cells from these toxins.

However, when microglia are activated by substances such as bacterial endotoxin, for example, lipopolysaccharide (LPS), interferon-γ, beta-amyloid or ganglioside, unlike normal microglia, they activate phagocytosis, proliferate and thus express cytokines such as TNF-α, IL-1β and IL-6, chemokines, and genes such as iNOS (inducible nitric oxide synthase) and COX-2 (cyclooxygenase-2) to produce inflammatory mediators. Such activation of microglia advantageously removes damaged cells and protects nerve cells from foreign bacteria or viruses, but nitric oxide (NO) produced by iNOS, prostaglandins produced by COX-2, and TNF-α and the like are toxic to nerve cells, thus aggravating damage to nerve cells. Therefore, proper suppression of the activation of microglia may be another approach to treat degenerative brain diseases.

In addition, astrocytes play an important role in maintaining normal brain activity as well as brain development. Astrocytes in the brain have been found to function to assist the activity of nerve cells while properly removing neurotransmitters secreted by nerve cells or regulating ion concentration in the brain. Further, astrocytes in the brain have been found to play a crucial role in the differentiation of neural stem cells into neurons.

However, upon injury to the brain, astrocytes proliferate actively, swell and are activated to reactive astrocytes through astrogliosis. These reactive astrocytes have been found in AIDS-related dementia, brain damage, ischemic brain disease, and Alzheimer's disease. Therefore, continuous activation of astrocytes eventually leads to the death of neurons. Therefore, inhibition of proper activation of astrocytes can also be another approach to treat degenerative brain diseases.

In addition, it is known that degenerative brain diseases including Alzheimer's disease are caused by the accumulation of beta-amyloid. Thus, research on removing plaques formed by aggregation of these proteins is ongoing. Research showing that tau protein forms an inclusion body when it has an abnormal structure, resulting in degenerative brain disease, has recently been published. Thus, tau protein is emerging as a target for these diseases.

Meanwhile, therapies currently used to treat degenerative brain diseases include drug therapy, surgical therapy, and physical therapy. Drug therapy is generally based on the use of drugs to supplement dopamine that is lacking in the brain, to correct the imbalance of neurotransmitters caused by lack of dopamine, to prevent or delay the destruction of nerve cells and to control other symptoms such as depression. It is not yet known exactly which mechanism acts on signaling and toxicity in the degenerative brain diseases. Therefore, there is no fundamental and effective therapeutic agent for degenerative brain diseases.

Meanwhile, abemaciclib is a drug currently used as a therapeutic agent for breast cancer and is only known to have inhibitory activity against cyclin-dependent kinase (CDK) 4 and 6. The relevance of abemaciclib to degenerative brain diseases has not yet been researched to date.

### Disclosure

### Technical Problems

Accordingly, the present inventors have found that abemaciclib, known as a conventional therapeutic agent for breast cancer, is effective to inhibit neuroinflammation, to inhibit hyperphosphorylation of tau protein, to improve long-term memory, and to inhibit amyloid plaque formation and thus is useful as a potential therapeutic agent for preventing or treating neurodegenerative disease. Based thereon, the present inventors completed the present invention.

Therefore, it is one object of the present invention to provide a pharmaceutical composition for preventing or treating neurodegenerative disease containing abemaciclib or a pharmaceutically acceptable salt thereof as an active ingredient.

It is another object of the present invention to provide a pharmaceutical composition for preventing or treating cognitive impairment, learning disability or memory impairment containing abemaciclib or a pharmaceutically acceptable salt thereof as an active ingredient.

It is another object of the present invention to provide a health functional food for preventing or ameliorating degenerative brain diseases containing abemaciclib or a salt thereof as an active ingredient.

It is another object of the present invention to provide a health functional food for preventing or ameliorating cognitive impairment, learning disability or memory impairment containing abemaciclib or a salt thereof as an active ingredient.

It is another object of the present invention to provide a pharmaceutical composition for preventing or treating neuroinflammatory diseases containing abemaciclib or a pharmaceutically acceptable salt thereof as an active ingredient.

It is another object of the present invention to provide a health functional food for preventing or ameliorating neuroinflammatory diseases containing abemaciclib or a pharmaceutically acceptable salt thereof as an active ingredient.

### Technical Solution

In accordance with one aspect of the present invention, the above and other objects can be accomplished by the provision of a pharmaceutical composition for preventing or treating a degenerative brain disease comprising abemaciclib or a pharmaceutically acceptable salt thereof as an active ingredient.

In an embodiment, the composition may inhibit expression and production of inflammatory cytokine including COX-2, IL-1β, IL-6 or iNOS.

In an embodiment, the composition may inhibit activity of toll-like receptor 4 (TLR4) and reduces a level of p-STAT3.

In an embodiment, the composition may inhibit microglia or astrocyte activation, inhibit amyloid plaques, increase the number of dendritic spines, inhibit tau phosphorylation, or improve memory and cognition.

In an embodiment, the degenerative brain disease may be selected from the group consisting of Alzheimer's disease, Parkinson's disease, Huntington's disease, multiple sclerosis, multiple system atrophy, epilepsy, encephalopathy, and stroke.

In another aspect of the present invention, provided is a pharmaceutical composition for preventing or treating cognitive impairment, learning disability or memory impairment containing abemaciclib or a pharmaceutically acceptable salt thereof as an active ingredient.

In another aspect of the present invention, provided is a health functional food for preventing or ameliorating a degenerative brain disease containing abemaciclib or a salt thereof as an active ingredient.

In another aspect of the present invention, provided is a health functional food for preventing or treating cognitive impairment, learning disability or memory impairment comprising abemaciclib or a pharmaceutically acceptable salt thereof as an active ingredient.

In an embodiment, the abemaciclib or salt thereof may inhibit expression and production of inflammatory cytokine including COX-2, IL-1β, IL-6 or iNOS.

In an embodiment, the abemaciclib or salt thereof may inhibit activity of toll-like receptor 4 (TLR4) and reduce a level of p-STAT3.

In an embodiment, the abemaciclib or salt thereof may inhibit microglia or astrocyte activation, inhibit amyloid plaques, increase the number of dendritic spines, inhibit tau phosphorylation, or improve memory and cognition.

In an embodiment, the degenerative brain disease may be selected from the group consisting of Alzheimer's disease, Parkinson's disease, Huntington's disease, multiple sclerosis, multiple system atrophy, epilepsy, encephalopathy, and stroke.

In another aspect of the present invention, provided is a pharmaceutical composition for preventing or treating a neuroinflammatory disease containing abemaciclib or a pharmaceutically acceptable salt thereof as an active ingredient.

In an embodiment, the neuroinflammatory disease may be selected from the group consisting of neuroblastoma, Lou Gehrig's disease, Creutzfeldt-Jakob disease, post-traumatic stress disorder, depression, schizophrenia, and amyotrophic lateral sclerosis.

In another aspect of the present invention, provided is a health functional food for preventing or ameliorating a neuroinflammatory disease containing abemaciclib or a pharmaceutically acceptable salt thereof as an active ingredient.

In an embodiment, the neuroinflammatory disease may be selected from the group consisting of neuroblastoma, Lou Gehrig's disease, Creutzfeldt-Jakob disease, post-traumatic stress disorder, depression, schizophrenia, and amyotrophic lateral sclerosis.

### Advantageous Effects

The abemaciclib according to the present invention is highly effective in inhibiting an pro-inflammatory cytokine such as COX-2, IL-1β, IL-6 or iNOS in neurons, inhibiting TLR4 signaling and the level of p-STAT3 associated with the neuroinflammatory response, inhibiting the LPS-mediated microglia or astrocyte activation and suppressed amyloid plaques, increased dendritic spine number, inhibiting tau phosphorylation, and improving the long-term memory in mice with neurodegenerative diseases. Based thereon, the abemaciclib of the present invention is useful as an active ingredient of pharmaceuticals and health functional foods for preventing, ameliorating, or treating neurodegenerative diseases or neuroinflammatory diseases.

### Description of Drawings

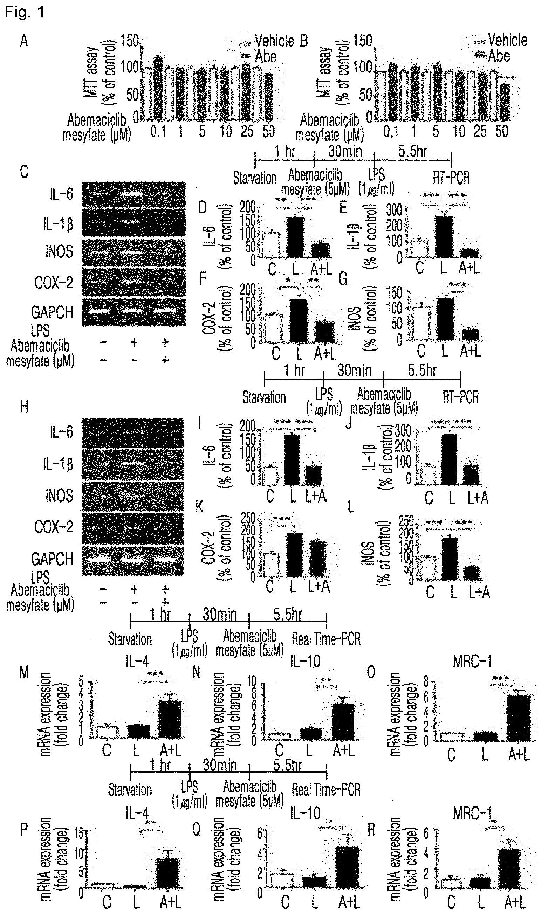

- FIG. 1: shows the result of analysis of the effects of treatment of microglial cells with abemaciclib on the levels of pro-inflammatory cytokines and anti-inflammatory cytokines induced by LPS, wherein (A) to (B) show the results of MTT assay after treatment with different concentrations of abemaciclib, (C) to (G) show the results of RT-PCR analysis of the levels of pro-inflammatory cytokines after pretreatment with abemaciclib, followed by treatment with LPS, (H) to (L) show the results of RT-PCR analysis of the levels of proinflammatory cytokines after pretreatment with LPS, followed by treatment with abemaciclib, and (M) to (O) show the results of Q-PCR analysis of the levels of anti-inflammatory cytokines after pretreatment with abemaciclib, followed by treatment with LPS, and (P) to (R) show the results of Q-PCR analysis of the levels of anti-inflammatory cytokines after treatment with LPS, followed by treatment with abemaciclib.

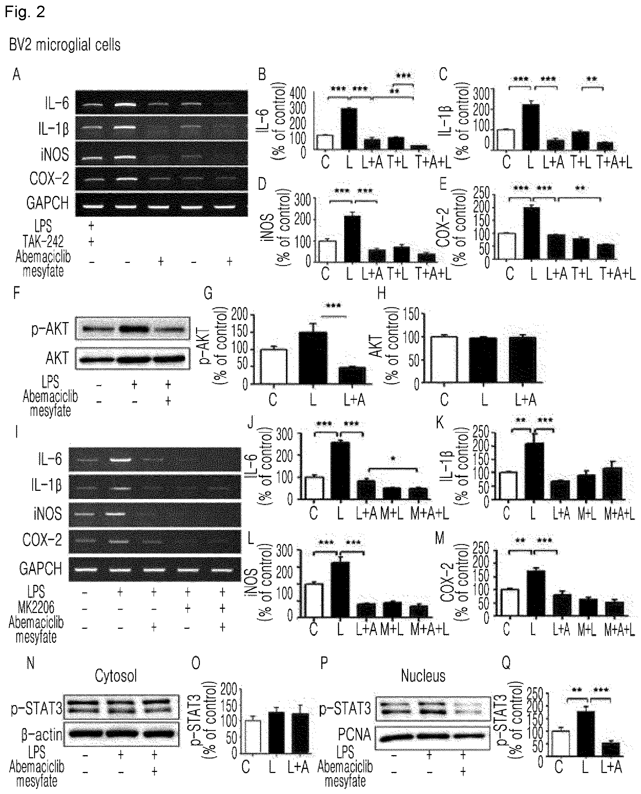

- FIG. 2: shows the result of determination as to whether or not the effect of abemaciclib on LPS-induced changes in pro-inflammatory cytokines depends on TLR4, wherein (A) to (E) show the levels of COX-2, IL-6, IL-1β and COX-2 in microglial cells measured by RT-PCR after treatment with TAK-242, abemaciclib and then LPS, (F) to (H) show the levels of p-AKT and AKT measured by Western blotting after treatment of microglia with abemaciclib and then LPS, (I) to (M) show the levels of COX-2, IL-6, IL-1β and COX-2 measured by RT-PCR after treatment of microglia with MK2206 (AKT inhibitor), abemaciclib, and then LPS, and (N) to (Q) show the level of p-STAT3 in each of nuclear and cytosolic fractions, measured by Western blotting, wherein the nuclear and cytosolic fractions are obtained from cell lysates obtained by treating microglia with abemaciclib and then LPS.

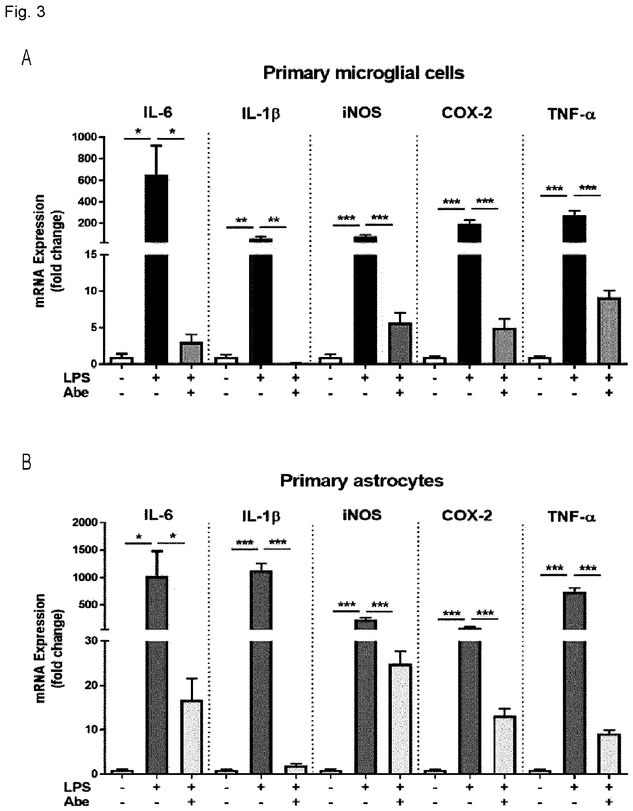

- FIG. 3: shows the results of Q-PCR analysis of the levels of pro-inflammatory cytokines after treating primary microglia and astrocytes with abemaciclib and then with LPS, wherein (A) shows the level of pro-inflammatory cytokine measured in primary microglia, and (B) shows the level of pro-inflammatory cytokine measured in primary astrocytes.

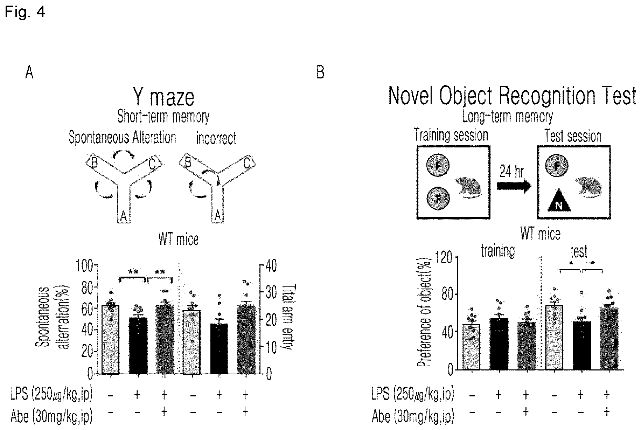

- FIG. 4: shows the result of behavioral experiments performed on days 8 and 9 when LPS was administered to wild-type mice 30 minutes after abemaciclib was administered thereto every day for 7 days in order to analyze the effect of abemaciclib on cognitive decline caused by LPS in wild-type mice, wherein (A) shows the result of Y maze analysis on day 8, and (B) shows the result of NOR test analysis on days 8 and 9.

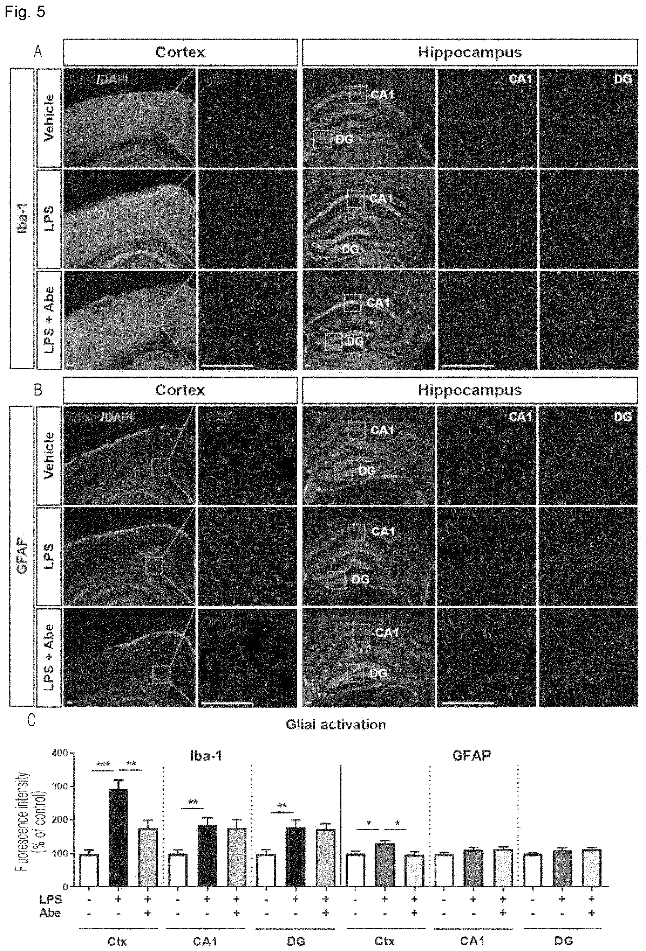

- FIG. 5: shows the results of immunohistochemistry performed in cortex and hippocampus of wild-type mice injected with abemaciclib daily for 3 days and then injected with LPS for 6 hours in order to analyze the effects of abemaciclib on LPS-induced inflammatory cytokine levels in wild-type mice, wherein (A) and (C) show the results when using anti-lba-1 antibody, and (B) and (C) show the results when using anti-GFAP antibody.

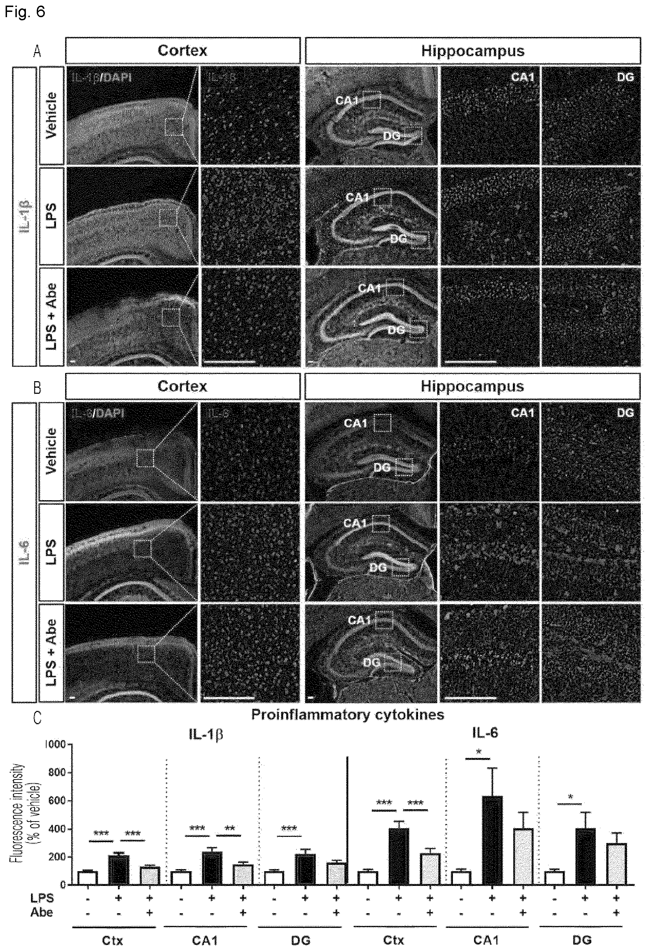

- FIG. 6: shows the results of immunohistochemistry performed in cortex and hippocampus of wild-type mice injected with abemaciclib mesylate daily for 3 days and then injected with LPS for 6 hours in order to analyze the effects of abemaciclib on LPS-mediated inflammatory cytokine IL-1β and IL-6 levels in wild-type mice, wherein (A) and (C) show the results when using anti-IL-1β antibody, and (B) and (C) show the results when using anti-IL-6 antibody.

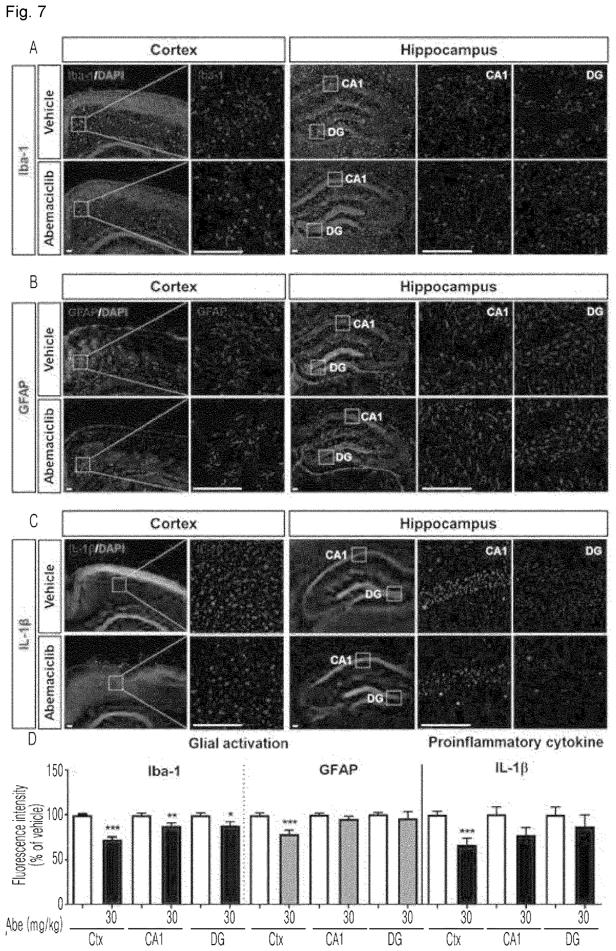

- FIG. 7: shows the results of immunohistochemistry performed in cortex and hippocampus of 5xFAD mice, an animal model of Alzheimer's disease treated with abemaciclib daily for 2 weeks, wherein (A) and (D) show the results when using anti-lba-1 antibody, (B) and (D) show the results when using anti-GFAP antibody, and (C) and (D) show the results when using anti-IL-1β antibody.

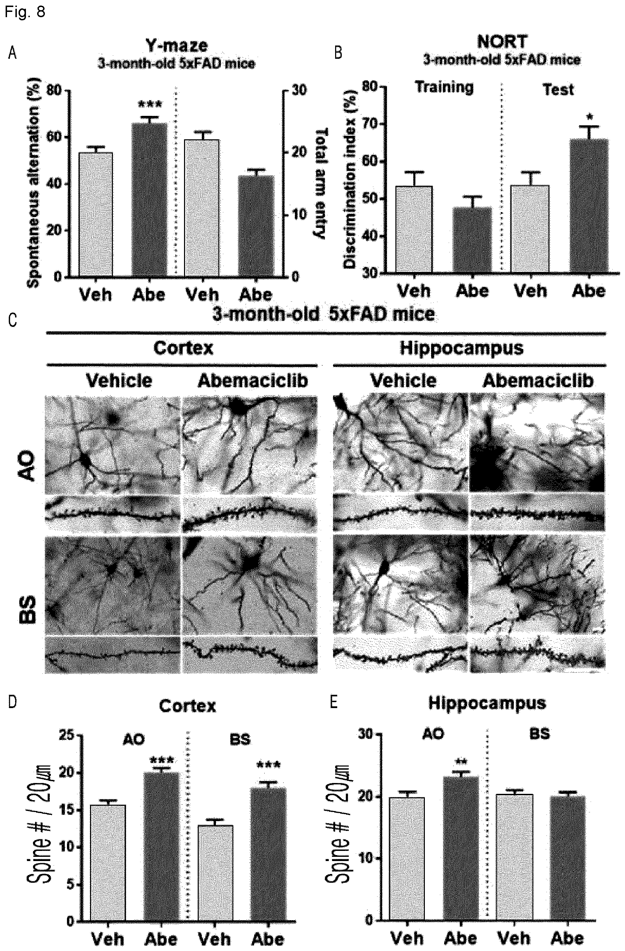

- FIG. 8: shows the results in 5xFAD mice, an animal model of Alzheimer's disease administered abemaciclib daily for 2 weeks on improvement in cognition and memory and increase dendritic spine number, compared to a vehicle-administered control group, wherein (A) to (B) shows the results of Y-maze, NOR training, and NOR test, and (C) to (E) show the number of dendritic spines in cortical and hippocampal tissues.

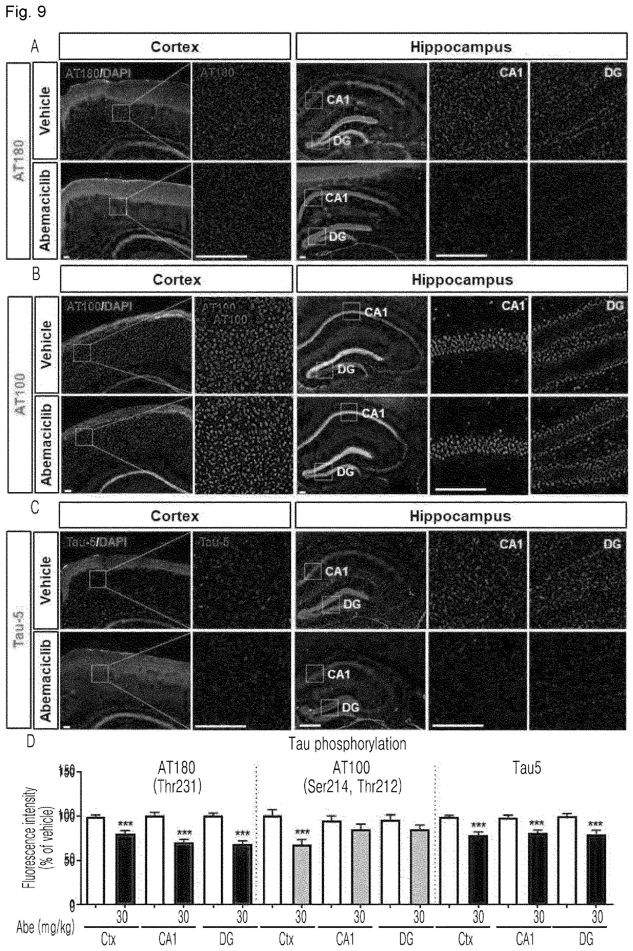

- FIG. 9: shows the Tau phosphorylation levels in cortical and hippocampal tissues of 5xFAD mice, an animal model of Alzheimer's disease administered abemaciclib, wherein (A) and (D) show the results of immunohistochemistry using anti-AT180 antibody, (B) and (D) show the results of immunohistochemistry using anti-AT100 antibody, and (C) and (D) show the results of immunohistochemistry using anti-tau5 antibody.

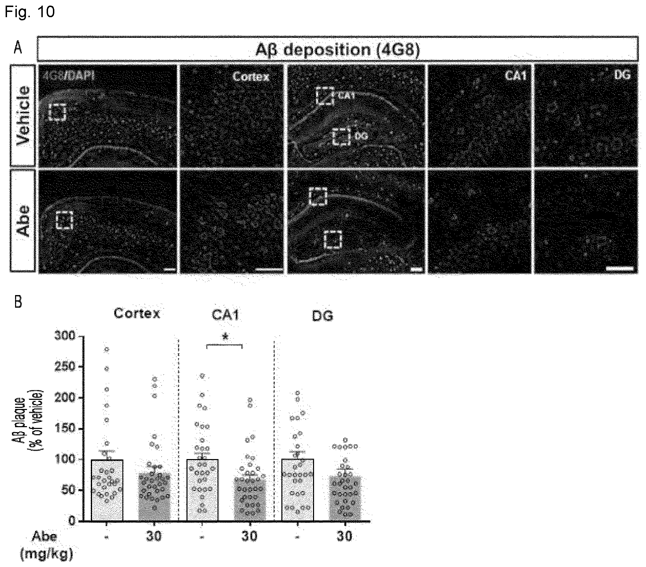

- FIG. 10: shows the results in 5xFAD mice, an animal model of Alzheimer's disease with abemaciclib on amyloid plaque inhibition in cortical and hippocampal tissues, wherein (A) and (B) show the result of immunohistochemistry using anti-4G8 antibody.

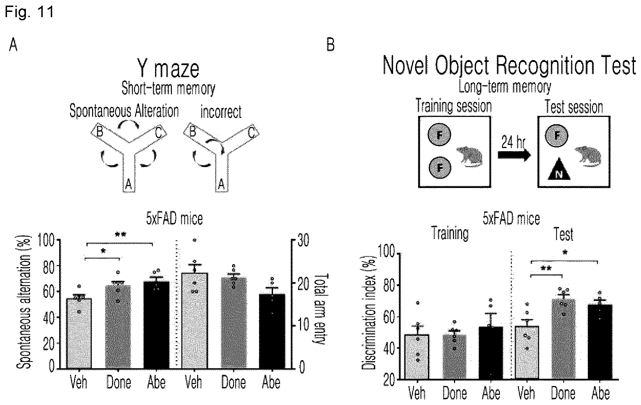

- FIG. 11: shows the analysis of cognition and memory improvement activities of 5xFAD mice, an animal model of Alzheimer's disease administrated abemaciclib or donepezil daily for 2 weeks and a control group administered a vehicle, wherein (A) shows the result of Y-maze and (B) shows the result of NOR training and NOR test.

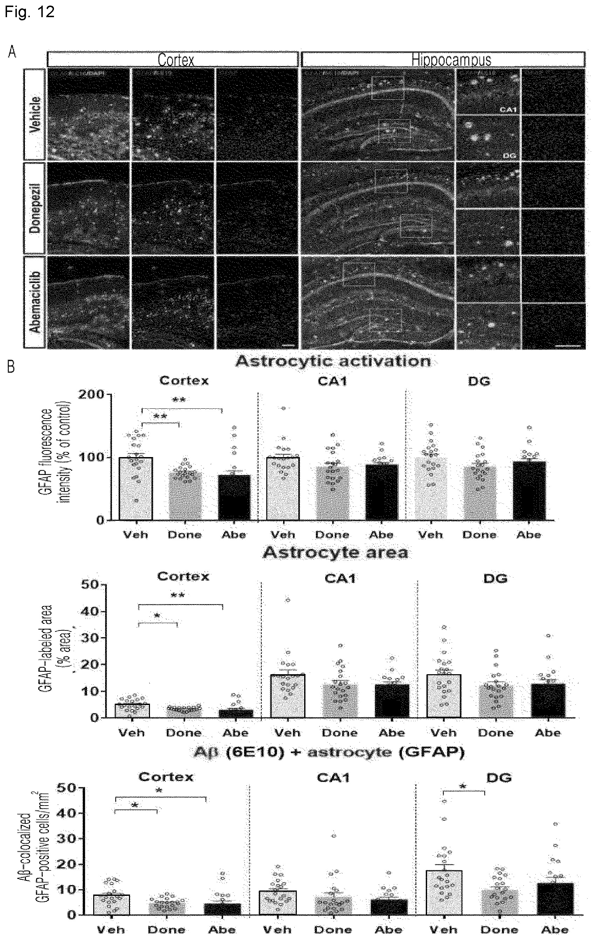

- FIG. 12: shows the results of immunohistochemistry of cortex and hippocampus of 5xFAD mice, an animal model of Alzheimer's disease administrated abemaciclib or donepezil daily for 2 weeks and a control group administered a vehicle, wherein (A) and (B) show the results of immunohistochemistry using anti-GFAP and anti-6E10 antibodies.

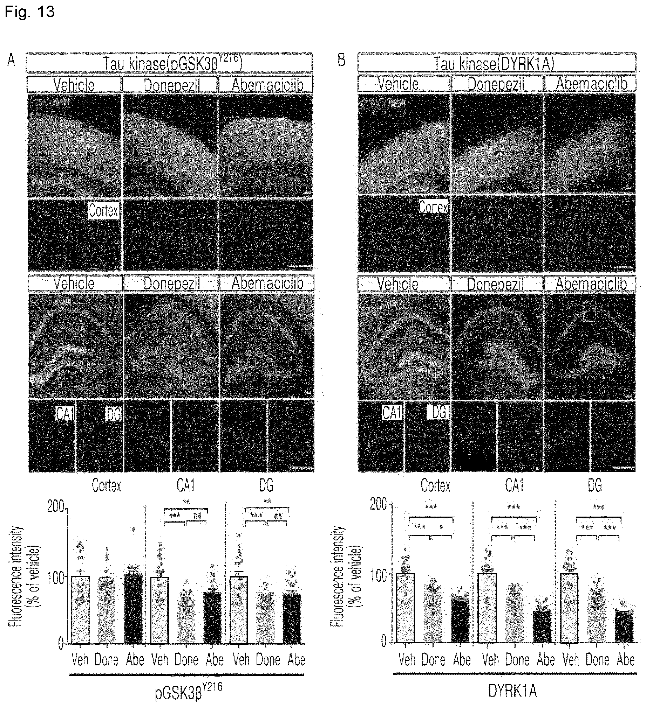

- FIG. 13: shows the result of immunohistochemistry of cortex and hippocampus of 5xFAD mice, an animal model of Alzheimer's disease administrated abemaciclib or donepezil daily for 2 weeks and a control group administered a vehicle, wherein (A) and (B) show the results of immunohistochemistry using anti-phospho-GSK-3β(Y216) or anti-DYRK1A antibodies.

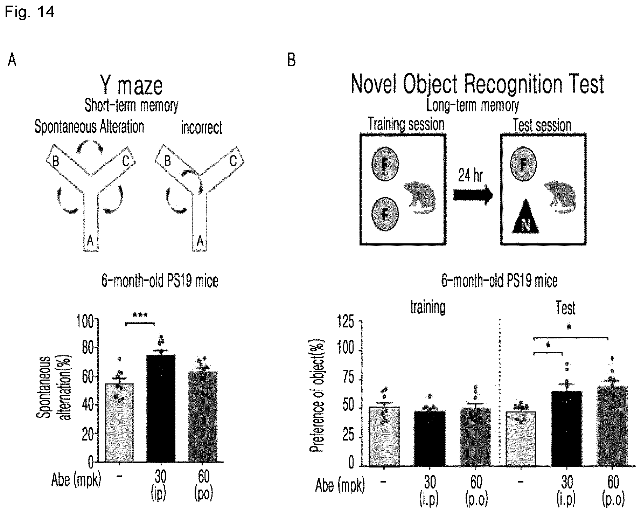

- FIG. 14: shows the improvement of cognition and memory of PS19 mice, an animal model of Alzheimer's disease administrated abemaciclib mesylate intraperitoneally (30 mg/kg) or orally (60 mg/kg) daily for 2 weeks and a control group administered a vehicle, wherein (A) shows the result of Y-maze test and (B) shows the result of analysis through NOR training and NOR test.

### Best Mode

The present invention provides a pharmaceutical composition for preventing or treating degenerative brain diseases containing abemaciclib or a pharmaceutically acceptable salt thereof as an active ingredient.

As described in Prior Art above, abemaciclib is known as a therapeutic agent for breast cancer, but has not yet been researched for the therapeutic potential thereof for degenerative brain diseases.

The abemaciclib according to the present invention may be used in the form of a pharmaceutically acceptable salt and an acid addition salt formed from a pharmaceutically acceptable free acid may be used as the salt. The acid addition salt is derived from inorganic acids such as hydrochloric acid, nitric acid, phosphoric acid, sulfuric acid, hydrobromic acid, hydroiodic acid, nitrous acid or phosphorous acid and non-toxic organic acids such as aliphatic mono- and di-carboxylates, phenyl-substituted alkanoates, hydroxyalkanoates, hydroxyalkanedioates, aromatic acids, aliphatic and aromatic sulfonic acids. The pharmaceutically non-toxic salt includes sulfate, pyrosulfate, bisulphate, sulphite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2-sulfonate or mandelate.

In one embodiment of the present invention, abemaciclib mesylate was used.

Accordingly, the present invention has identified, for the first time, that abemaciclib can be used as a therapeutic agent for neurodegenerative diseases.

According to one embodiment of the present invention, it was found that abemaciclib can effectively inhibit the expression and production of inflammatory cytokines and pro-inflammatory cytokines induced by LPS known as an inflammation-inducing factor in glial cells. Specifically, it was found that abemaciclib can effectively inhibit the production of COX-2, IL-1β, IL-6 or iNOS by LPS. Among various inflammation-inducing factors, LPS (lipopolysaccharide), which is one of the endotoxins, facilitates the secretion of pro-inflammatory cytokines, which leads to the secretion of inflammatory mediators such as nitric oxide and prostaglandins, and various inflammatory diseases due to excessive inflammatory reactions.

Accordingly, the present inventors found that abemaciclib can effectively inhibit the LPS-induced proinflammatory response of abemaciclib and thus is capable of preventing, ameliorating, or treating inflammatory diseases and brain inflammatory diseases which may be caused by excessive inflammatory reactions.

In another embodiment of the present invention, an experiment was performed to determine whether inhibition of inflammatory cytokines by abemaciclib is related to the TLR4/AKT/STAT3 signaling system.

TLR4 is known to induce the pathology of inflammatory diseases due to the generation of various inflammatory factors caused by NF-kB activated upon stimulation of cells by LPS, and AKT is known to activate NF-kB. It is known that signaling of AKT is known to be related to the immune response induced by LPS through the TLR4 signaling pathway and the level of pro-inflammatory cytokines is reduced in human liver cancer cell lines when AKT is knocked out. In addition, it is known that activated TLR4 affects STAT3 and other transcription factors and continuously activated STAT3 is closely related to the development of inflammatory diseases.

According to one embodiment of the present invention, it was found that abemaciclib inhibits TLR4/AKT/STAT3 activation, and the levels of LPS-induced inflammatory cytokines such as COX-2, IL-1β, IL-6 and iNOS are decreased in both microglia and astrocytes by treatment with abemaciclib and the AKT phosphorylation was also reduced.

Therefore, it was found that abemaciclib can inhibit TLR4/AKT/STAT3 activation involved in induction of inflammation in brain neurons.

In addition, abemaciclib of the present invention was found to have an effect of inhibiting damage to nerve cells caused by activated microglia or astrocytes by inhibiting the activity of microglia or astrocytes caused by LPS.

Microglia, which act as macrophages in the brain, are essential effector cells that regulate immune responses in the central nervous system (CNS). The activation thereof plays an important role in maintaining CNS homeostasis by removing foreign substances caused by drugs or toxins and secreting nerve growth factors. However, when exposed to harmful stresses such as signals generated from damaged neurons, accumulation of abnormal proteins modified by external stimuli, and invasion of pathogens, the activity of microglia is excessively increased, causing damage to neurons, resulting in degenerative neurological diseases such as Alzheimer's disease, Parkinson's disease, multiple sclerosis, and cerebral infarction. In addition, recent research has revealed that an excessive inflammatory response in microglia or astrocytes causes neurodegenerative diseases.

Specifically, unlike normal microglia, excessively activated microglia actively perform phagocytosis, proliferate and express cytokines such as TNF-α, IL-113, and IL-6, genes such as chemokines, and iNOS, inducible nitric oxide synthase (COX-2) and cyclooxygenase-2 (COX-2) to produce inflammatory mediators. Activation of microglia advantageously removes damaged cells and protects neurons from invading bacteria or viruses, but disadvantageously aggravates damage to nerve cells and causes degenerative brain diseases or neurodegenerative diseases because nitric oxide (NO) produced by iNOS and prostaglandins produced by COX-2, TNF-α and the like are toxic to nerve cells.

In addition, astrocytes are also known to play an important role in maintaining normal brain activity. In particular, astrocytes are known to play a role in synapse formation of nerve cells, regulation of synapse number, synaptic functions, and differentiation of neural stem cells into neurons. However, when these astrocytes are excessively reactive, that is, when they remain excessively activated, they cause the death of neurons and also induce the death of neighboring neurons, thus causing degenerative brain diseases. Therefore, inhibition of excessive activation of astrocytes may also be a novel approach to treat degenerative brain diseases.

Substances that cause excessive activation of microglia and astrocytes include bacterial endotoxin lipopolysaccharide (LPS), interferon-γ, beta amyloid, ganglioside, and the like, which can induce an inflammatory response.

In addition, in another embodiment of the present invention, it was found that abemaciclib can increase dendritic spine number in hippocampal neurons, and furthermore, administration of abemaciclib to mice with Alzheimer's disease, which is a degenerative brain disease, results in activities to inhibit amyloid plaque accumulation, beta amyloid, and Tau phosphorylation.

Beta amyloid (Aβ: amyloid beta) is derived from amyloid precursor protein (APP), which is degraded by beta secretase and gamma secretase to produce Aβ, is a main component of amyloid plaques often found in the brains of patients with brain diseases such as Alzheimer's disease and causes various brain diseases when it accumulates abnormally.

In addition, excessive accumulation of beta-amyloid causes an inflammatory response in the hippocampus, cerebral cortex, and peritoneal cells, thus causing damage to nerve cells as well as the neural network that maintains the normal function of the brain.

In addition, tau protein is involved in the entanglement of nerve fibers with a schedule of microtubule-binding proteins having a molecular weight of 50,000 to 70,000 Da. Tau protein is one of the microtubule-associated proteins present in the axon of normal neurons. Excessive accumulation of these tau proteins causes the microtubules to collapse and breakage of the network of normal nerve cells. It is known that the accumulation of tau protein is caused by excessive phosphorus attachment to tau protein to form neurofibrillary tangles due to phosphorylation, and the accumulation of these tau proteins and the aggregation of nerve cells cause various degenerative brain diseases.

In this regard, abemaciclib of the present invention has inhibitory activity against beta amyloid and Tau phosphorylation and thus can be used as a therapeutic agent for neurodegenerative diseases caused by Tau phosphorylation in nerve cells or brain cells.

In addition, in one embodiment of the present invention, Y-maze and NOR tests for cognitive and memory analysis were performed on mice of Alzheimer's disease in a group administered abemaciclib and vehicle-administered group. It was found that mice of Alzheimer's disease treated with abemaciclib exhibited improved long-term memory compared to control group mice.

Therefore, abemaciclib of the present invention can be used as a therapeutic agent for preventing, ameliorating, or treating degenerative brain diseases, and the degenerative brain diseases may be selected from the group consisting of Alzheimer's disease, Parkinson's disease, Huntington's disease, multiple sclerosis, multiple system atrophy, epilepsy, encephalopathy, and stroke, but are not limited thereto.

Furthermore, it was found that abemaciclib of the present invention has effects of improving memory, cognitive ability, and learning ability in a mouse animal model in which degenerative brain disease was induced, and thus the composition containing abemaciclib or a pharmaceutically acceptable salt thereof according to the present invention as an active ingredient can also be used as a pharmaceutical composition for preventing or treating cognitive impairment, learning disability or memory impairment.

As used herein, the term "improvement of memory" and "improvement of cognitive ability" mean prevention of memory decline, memory impairment or cognitive ability deterioration that is caused by brain atrophy and destruction of brain nerve cells due to physical fatigue, lack of sleep, excessive alcohol intake, dementia, or the like, wherein the cognitive ability is maintained by controlling harmful substances that damage brain cells or is improved by regulating neurotransmitters in the brain. Memory refers to the ability to accept necessary information, store the information in the brain, and then use the information when necessary, and cognitive ability refers to the ability to recognize and distinguish objects.

The pharmaceutical composition of the present invention may include a pharmaceutically acceptable carrier. The composition containing a pharmaceutically acceptable carrier may be selected from various oral or parenteral formulations. In this regard, the pharmaceutical composition may be formulated using an ordinary diluent or excipient such as a filler, a thickener, a binder, a wetting agent, a disintegrant, a surfactant, or the like.

Solid formulations for oral administration may include tablets, pills, powders, granules, capsules and the like. Such a solid formulation is prepared by mixing at least one compound with at least one excipient such as starch, calcium carbonate, sucrose, lactose or gelatin. In addition to a simple excipient, a lubricant such as magnesium stearate or talc may be further used. Liquid formulations for oral administration may include suspensions, solutions for internal use, emulsions, syrups, and the like. In addition to a simple diluent such as water or liquid paraffin, various excipients such as wetting agents, sweeteners, aromatics and preservatives may be incorporated in the liquid formulations.

In addition, formulations for parenteral administration include sterile aqueous solutions, non-aqueous solvents, suspensions, emulsions, lyophilizates, suppositories and the like. Useful non-aqueous solvents and suspensions include propylene glycol, polyethylene glycol, vegetable oils such as olive oil and injectable esters such as ethyl oleate. The base ingredients of suppositories include Witepsol, macrogol, Tween 61, cacao butter, laurin butter and glycerogelatin.

The pharmaceutical composition is selected from the group consisting of tablets, pills, powders, granules, capsules, suspensions, solutions for internal use, emulsions, syrups, sterilized aqueous solutions, non-aqueous solutions, suspensions, emulsions, lyophilizates and suppositories.

In addition, as described above, these pharmaceutical compositions of the present invention may be administered to treat various diseases including symptoms associated with degenerative brain diseases.

The pharmaceutical composition according to the present invention may be administered in a pharmaceutically effective amount. The term "pharmaceutically effective amount" refers to an amount sufficient for treating a disease at a reasonable benefit/risk ratio applicable to all medical treatments. The effective amount may be determined depending on a variety of factors including the type, severity, age, and gender of the subject, drug activity, sensitivity to drugs, administration time, route of administration and excretion rate, duration of treatment, and drug used in combination, and other factors well-known in the pharmaceutical field.

In addition, the composition of the present invention may be administered alone or in combination with other therapeutics. In this case, the composition may be administered sequentially or simultaneously with conventional therapeutics. In addition, the composition may be administered in a single or multiple doses. Taking into consideration these factors, it is important to administer the composition in the minimum amount sufficient to achieve maximum efficacy without side effects. A general dosage of the pharmaceutical composition of the present invention is within the range of 0.001 to 100 mg/kg for an adult.

The pharmaceutical composition according to the present invention may be administered through any one of various routes enabling the composition to be delivered to a target tissue. The composition of the present invention may be administered intraperitoneally, intravenously, intramuscularly, subcutaneously, intradermally, orally, intranasally, intrapulmonarily, or intrarectally, as desired, but is not limited thereto. In addition, the pharmaceutical composition may be administered using any device capable of delivering the active substance to target cells.

The composition of the present invention may be used alone or in combination with another method such as surgery, hormone therapy, drug therapy, and biological response modifier-based method for preventing and treating inflammatory diseases.

Furthermore, the present invention provides a health functional food for preventing or ameliorating degenerative brain diseases containing abemaciclib mesylate as an active ingredient.

Furthermore, the present invention provides a health functional food for preventing or ameliorating cognitive impairment, learning disability or memory impairment containing abemaciclib mesylate as an active ingredient.

The health functional food of the present invention may further include a cytologically acceptable carrier.

The health functional food includes a formulation such as a pill, powder, granule, infusion, tablet, capsule, or liquid and there is no particular limitation as to the type of food to which the active ingredient of the present invention can be added. Examples of the food include various beverages, chewing gums, tea, vitamin complexes, health supplements and the like.

In addition to the active ingredient of the present invention, the health functional food may further include other ingredients that do not interfere with the prevention and amelioration of degenerative brain diseases and the type thereof is not particularly limited. For example, like conventional foods, the health functional food may further include various herbal extracts, cytologically acceptable food additives, or natural carbohydrates as additional ingredients.

As used herein, the term "health functional food" refers to food manufactured and processed in the form of tablets, capsules, powders, granules, liquids and pills using raw materials or ingredients having functionalities beneficial to the human body. Here, the term "functional" refers to adjusting nutrients for the structure and function of the human body or obtaining useful effects for health purposes such as physiological functions. The health functional food of the present invention may be prepared by a method commonly used in the art and may be prepared by adding raw materials and ingredients commonly added in the art during the preparation. In addition, unlike general drugs, the health functional food uses food as a raw material and thus has advantages of causing no side effect when taking a drug for a long time and exhibiting excellent portability.

There is no particular limitation as to the type of health functional food of the present invention, and specific examples thereof include meat, sausage, bread, chocolate, candy, snacks, confectionery, pizza, ramen, other noodles, gum, dairy products including ice cream, various soups, beverages, tea, drinks, alcoholic beverages, vitamin complexes, and the like. The health functional food of the present invention may include all health functional foods in a conventional sense.

Furthermore, the present invention provides a pharmaceutical composition for preventing or treating neuroinflammatory diseases containing abemaciclib or a pharmaceutically acceptable salt thereof as an active ingredient.

The neuroinflammatory response involves activation of innate immune cells (microglia), release of inflammatory mediators such as nitric oxide (NO), cytokines and chemokines and infiltration of macrophages, leading to neuronal cell death. Inflammatory activation of microglia and astrocytes is considered as a pathological marker and an important mechanism in the progression of neurodegenerative diseases. Strict regulation of microglia activity is essential for maintaining brain homeostasis and preventing infectious and inflammatory diseases.

Meanwhile, in one embodiment of the present invention, it was found that abemaciclib can effectively inhibit LPS-induced pro-inflammatory cytokine levels, and increase the expression of anti-inflammatory factors in microglia and astrocytes. In addition, it was found through experiments that abemaciclib can effectively inhibit LPS-mediated microglia and astrocytes activation

Therefore, abemaciclib of the present invention has effects of suppressing neuroinflammation in brain cells and preventing or treating neuroinflammatory diseases through inhibition of the activity of microglia and astrocytes.

In the present invention, the neuroinflammatory disease may be selected from the group consisting of, but not limited to, neuroblastoma, Lou Gehrig's disease, Creutzfeldt-Jakob disease, post-traumatic stress disorder, depression, schizophrenia, and amyotrophic lateral sclerosis.

In addition, the present invention can provide a health functional food for preventing or ameliorating neuroinflammatory diseases containing abemaciclib or a pharmaceutically acceptable salt thereof as an active ingredient.

### Mode Invention

Hereinafter, the present invention will be described in more detail with reference to examples. However, it will be obvious to those skilled in the art that these examples are provided only for illustration of the present invention, and should not be construed as limiting the scope of the present invention.

### <Preparation example and experiment method>

All experiments in Examples of the present invention were performed with the approval (KBRI, approval numbers 19-00049 and 19-00042) of the Animal Experimentation Ethics Committee (IACUC) of the Korea Brain Research Institute.

### Cell lines and culture conditions

BV2 microglial cells (obtained from Dr. Kyung-Ho Suk) were cultured in DMEM/high glucose (Invitrogen, Carlsbad, CA, USA) supplemented with 5% fetal bovine serum (FBS; Invitrogen, Carlsbad, CA, USA) in a 5% CO2 incubator. In addition, the results obtained from all in vitro experiments were analyzed semi-automatically using Image J software and the results were confirmed by an independent researcher who did not participate in the current experiment. In addition, commercially available abemaciclib mesylate was used as abemaciclib in the following examples of the present invention.

### MTT assay

Cell viability was measured using the 3-(4,5-dimethylthiazol-2-yl)-2,5-diphenyltetrazolium bromide (MTT) assay. BV2 microglia were seeded in a 96-well plate and treated with various concentrations of abemaciclib mesylate (0.1, 1, 5, 10, 25, 50 µM) for 24 hours in the absence of FBS. Then, the cells were treated with 0.5 mg/ml MTT and incubated at 37°C for 3 hours in a 5% CO2 incubator, and absorbance was measured at 580 nm.

### Reverse transcription-polymerase chain reaction (RT-PCR)

Total RNA was extracted from cells using TriZol (Invitrogen). Then, total RNA was reverse-transcribed into cDNA using Superscript cDNA Premix Kit II with oligoDT (GeNetBio, Korea). RT-PCR was performed with Prime Taq Premix (GeNetBio, Korea) using the following primers for BV microglia.
IL-1β Forward (F): 5'-AGC TGG AGA GTG TGG ATC CC-3'
IL-1β Reverse (R): 5'-CCT GTC TTG GCC GAG GAC TA-3'
IL-6 Forward (F): 5'-CCA CTT CAC AAG TCG GAG GC-3'
IL-6 Reverse (R): 5'-GGA GAG CAT TGG AAA TTG GGG T-3'
COX-2 Forward (F): 5'-GCC AGC AAA GCC TAG AGC AA-3'
COX-2 Reverse (R): 5'-GCC TTC TGC AGT CCA GGT TC-3'
iNOS Forward (F): 5'-CCG GCA AAC CCA AGG TCT AC-3'
iNOS Reverse (R): 5'-GCA TTT CGC TGT CTC CCC AA-3'
GAPDH Forward (F): 5'-CAG GAG CGA GAC CCC ACT AA-3'
GAPDH Reverse (R): 5'-ATC ACG CCA CAG CTT TCC AG-3'

Then, the RT-PCR products were separated by electrophoresis on a 1.5% agarose gel containing Eco Dye (1:5000, Korea). The electrophoresed RT-PCR products were image-analyzed using Image J (NIH) and Fusion (Korea).

### Real-time PCR (Q-PCR)

In order to determine the effect of abemaciclib mesylate on the levels of LPS-induced proinflammatory and anti-inflammatory cytokines, BV2 microglia and mouse primary microglia were treated with LPS (1 µg/ml) or PBS for 30 minutes, and further treated with abemaciclib mesylate (5 µM) or vehicle (1% DMSO) for 5.5 hours. At this time, in another experiment, BV2 microglia and mouse primary microglia were first treated with abemaciclib mesylate and then with LPS.

Then, total RNA was extracted using TRIzol (Invitrogen) according to the manufacturer's protocol and reverse-transcribed into cDNA using Superscript cDNA Premix Kit II (GeNetBio, Korea) including oligo (dT) primers. Then, real-time PCR was performed using Fast SYBR Green Master Mix (Thermo Fisher Scientific, CA, USA) and QuantStudio 5 Real-Time PCR system (Thermo Fisher Scientific, San Jose, CA, USA). Cycle threshold (Ct) for mRNA of inflammatory mediators were normalized to Ct for Gapdh and data were quantified as fold change relative to control group.

### Antibodies and Inhibitors

Primary antibodies used for Western blot and immunohistochemistry (IHC) were rabbit anti-AKT (1:1000 for WB, Santa Cruz Biotechnology), rabbit anti-p-AKT (Ser473) (1:1000 for WB, Cell Signaling Technology), rabbit anti-STAT3 (Y705, 1:1000 for WB, Cell Signaling Technology), rabbit anti-lba-1 (1:500 for IHC, Wako, Japan), rabbit anti-GFAP (1:500 for IHC, Neuromics, U.S.A), rabbit anti-IL-1beta (1:100 for IHC, Abcam, UK), mouse anti-IL-6 (1:100 for IHC, Santacruz Technology, U.S.A), mouse anti-AT100 (1:200, Invitrogen, U.S.A), mouse anti-AT180 (1:200, Invitrogen, U.S.A), mouse anti-Tau5 (1:200, Invitrogen, U.S.A), rabbit anti-DYRK1A (1:200, Abcam, UK), mouse anti-4G8 (1:500, Biolegend, U.S.A), mouse anti-6E10 (1:500, Biolegend, U.S.A), and rabbit anti-phospho-GSK-3β(Y216) (1:200, Abcam). Also, inhibitors used therefor include TLR4 inhibitor (TAK-242, 500 nM; Calbiochem), and AKT inhibitor (MK2206, 10 mM; Selleckchem). In addition, LPS, which was derived from Escherichia coli O111:B4, was purchased from Sigma-Aldrich (St. Louis, MO, USA).

### Western blotting

In order to determine whether or not abemaciclib mesylate affects AKT signaling, BV2 microglia were treated with abemaciclib mesylate (5 µM) or vehicle (1% DMSO) for 45 min, followed by treatment with LPS (1 µg/ml) or PBS for 45 minutes.

Then, the cells were lysed using RIPA buffer containing protease and phosphatase inhibitors (Roche, USA). Western blotting was performed by conventional Western blotting methods and image analysis was performed using Fusion software.

### Cytosolic and nuclear fractionation

In order to determine whether or not abemaciclib mesylate affects the level of p-STAT3 in the cytoplasm and nucleus induced by LPS, BV2 microglia were incubated in serum free media for 1 hour and treated with abemaciclib mesylate (5 µM) or vehicle (1% DMSO) for 30 minutes, followed by treatment with LPS (1 µg/ml) or PBS for 5.5 hours. Then, the cells were lysed using a buffer for cytosolic fractionation (10 mM HEPES pH 8.0, 1.5 mM MgCl2, 10 mM KCI, 0.5 mM DTT, 300 mM sucrose, 0.1% NP-40 and 0.5 mM PMSF). 5 minutes after the solution was added, the cell lysate was centrifuged at 10,000 rpm and 4°C for 1 minute and the supernatant was separated into a cytosolic fraction and stored. The pellet was lysed in nuclear fractionation buffer (10 mM HEPES pH 8.0, 20% glycerol, 100 mM KCI, 100 mM NaCl, 0.2 mM EDTA, 0.5 mM DTT and 0.5 mM PMSF) on ice for 15 minutes Subsequently, the result was centrifuged at 4°C at 10,000 rpm for 15 minutes. Western blotting was performed using anti-STAT3 (Y705), PCNA, and β-actin antibodies and analyzed using Fusion software.

### Culture of mouse primary microglia and astrocytes

In order to determine whether or not abemaciclib mesylate modulates the levels of LPS-induced inflammatory cytokines in primary glial cells, a mouse mix culture (mixture of primary microglia and primary astrocytes) was prepared. Specifically, whole brains from postnatal 1-day-old C57BL/6 mice were minced and mechanically disrupted using a 70 µm nylon mesh. Mixed glial cells were seeded into 75 T culture flasks and grown in low glucose DMEM medium supplemented with 10% FBS (containing 100 units/mL penicillin and 100 µg/mL streptomycin). Then, in order to obtain mouse primary astrocytes, the mixed glial cells were shaken overnight at 250 rpm. The next day, the conditioned medium was discarded and the remaining cells were detached using trypsin-EDTA and centrifuged (1,200 rpm, 30 minutes). After centrifugation, the conditioned medium was removed and cell pellets (primary astrocytes) were collected to analyze the effect of abemaciclib mesylate on neuroinflammation in primary astrocytes.

In order to obtain mouse primary microglia, the mixed glial cells were subjected to mild trypsinization (5-fold dilution of 0.25% trypsin in serum-free DMEM) in a 5% CO2 incubator at 37°C for 40 minutes. Then, the upper astrocyte layer was discarded, low-glucose DMEM containing 10% FBS was added, and the remaining primary microglia were used in the experiment. Microglia or astrocytes were cultured in serum free media for 1 hour, treated with abemaciclib (5 µM) or vehicle (1% DMSO) for 30 minutes, and then treated with LPS (200 ng/ml) or PBS for 5.5 hours, and real-time PCR was performed.

### Wild type mice

All experiments were performed in accordance with animal experiments and guidelines approved by the Korea Brain Research Institute (IACUC-2016-0013). C57BL6/N mice purchased from OrientBio Company were used and male C57BL6/N (8 weeks, 25-30 g) mice were bred in a pathogen-free facility under a 12-hour light/dark cycle at an ambient temperature of 22°C. To determine the effect of abemaciclib on LPS-induced cognitive dysfunction, wild-type mice were administered abemaciclib (30 mg/kg, intraperitoneal administration) or vehicle (10% DMSO) daily for 7 days, 30 minutes later, the mice were administered LPS (250 µg/ml, intraperitoneal administration) or PBS, and Y maze and NOR tests were performed on days 8 and 9. During the behavioral test period (8 and 9 days), abemaciclib and LPS were administered after the behavioral test. In order to determine the effect of abemaciclib mesylate on LPS-induced neuroinflammation, wild-type mice were intraperitoneally administered abemaciclib mesylate (30 mg/kg, i.p.) or vehicle (10% DMSO) daily for 3 days. Then, LPS (Sigma, Escherichia coli, 10 mg/kg, i.p.) or PBS was continuously administered thereto for 6 hours and then brain tissue was analyzed. Data were also analyzed in a semi-automated manner using Image J software, and the results were confirmed by an independent researcher who did not particulate in the current experiment.

### Production of animal model of Alzheimer's disease (5x FAD mice)

F1-generation male 5xFAD mice (stock# 34848-JAX, B6Cg-Tg APPSwFILon, PSEN1 * M146L * L286V6799Vas/Mmjax) purchased from Jackson Laboratory were used. 5xFAD mice (3 months old) were bred in a sterile facility under 12-hour light/dark controlled conditions at an ambient temperature of 22°C. After daily administration of abemaciclib (30 mg/kg, intraperitoneal administration) or vehicle (10% DMSO) to 5xFAD mice for 14 days, Y maze test on day 15 and NOR test on day 16 to 17 were performed, followed by fluorescent tissue staining or Golgi staining. In addition, in order to compare the effect of abemaciclib on regulation of degenerative brain disease symptoms with that of donepezil, a currently marketed therapeutic agent for dementia, abemaciclib (30 mg/kg, intraperitoneal administration) and donepezil (1 mg/kg), intraperitoneal administration) or vehicle (10% DMSO) was administered daily to 5xFAD mice for 14 days, followed by Y maze test on day 15 and NOR test on days 16 to 17, followed by fluorescent tissue staining.

### Production of animal model of Alzheimer's disease(PS19 mice)

Male PS19 mice (stock # 008169-JAX, B6;C3-Tg (Prnp-MAPT * P301S) PS19Vle/J) purchased from Jackson Laboratory were used. PS19 mice (6 months old) were bred in a sterile facility at an ambient temperature of 22°C for a 12-hour shift of the light-dark cycle. After daily administration of abemaciclib (30 mg/kg, peritoneal administration or 60 mg/kg, oral administration) or vehicle (10% DMSO) to the PS19 mice for 14 days, Y maze test on day 15 and NOR test on days 15 to 16 were performed.

### Immunofluorescence staining

In order to determine the effect of abemaciclib mesylate on LPS-induced neuroinflammation in vivo, abemaciclib mesylate (30 mg/kg, i.p.) or vehicle (10% DMSO) was intraperitoneally administered to wild-type mice daily for 3 days, followed by continuous administration of LPS (Sigma, Escherichia coli, 10 mg/kg, ip) or PBS for 6 hours. After injection of LPS or PBS, the mice were perfused and fixed with 4% paraformaldehyde (PFA) solution, and then the brain tissue was flash-frozen and sectioned using a cryostat (35 µm thickness). For another immunohistochemical experiment, abemaciclib mesylate (30 mg/kg, i.p.) or vehicle (10% DMSO) was intraperitoneally administered daily for 2 weeks to Alzheimer's-induced mice (5x FAD mice), and 14 days later, as described above, brain tissue was sectionized in the same manner as above.

Then, each brain section was subjected to immunohistochemical staining. Brain sections were rinsed in PBS and permeabilized for 1 hour at room temperature using PBS containing 0.2% Triton X-100 and 1% BSA. Then, the primary antibody was diluted in PBST (0.2% Triton X-100 in PBS) and reacted at 4°C for 24 hours, and the next day, the tissue was washed three times with PBST, and then reacted with a secondary antibody bound with a fluorescent signal at room temperature for 2 hours. Then, the brain sections were washed three times with PBS, placed on slides, and observed under a fluorescence microscope.

### Golgi staining

In order to determine the effect of abemaciclib mesylate on dendritic spinogenesis in vivo, Golgi staining was performed using the FD Rapid GolgiStain Kit (FD Neurotechnologies, Ellicott City, MD, USA). Specifically, animals injected with vehicle or abemaciclib mesylate were immersed in solutions A and B, respectively, for 15 days in the dark, and then transferred to solution C and allowed to stand for 24 hours. After 24 hours, the solution C was replaced with fresh one, and the brain of each mouse was sliced to a thickness of 150 µm using a VT1000S Vibratome (Leica, Bannockburn, IL, USA). Dendritic images were obtained with a bright field microscope using Axioplan 2 (Zeiss, Oberkochen, Germany). Dendritic spine density was measured using 8 to 10 sections of each mouse brain ranging from -1.70 mm to -2.30 mm relative to bregma. All data were analyzed using Image J software and all test results were verified by an independent researcher who did not participate in the current experiment.

### Y maze test

In order to verify the effects of administration of abemaciclib mesylate to normal animals (wild-type mice) or Alzheimer's-induced animal models (5xFAD mice and PS19 mice) on the improvement of short-term memory, each arm is 42 cm long, 3 cm wide, and 12 cm high, and the animal was placed at the end of one arm of a Y-shaped maze having three arms forming at an angle of 120 degrees, and the arm of the mouse visited was recorded for 5 minutes. Then, percent alteration = (number of alterations/number of triads) x 100 was obtained and used as a parameter of short-term memory.
Alteration: 1 point awarded when the mouse enters three different arms in turn
Triads: Total visits - 2

### Novel object recognition test

In order to verify the effect of administration of abemaciclib mesylate to normal animals (wild-type mice) or animal model of Alzheimer's disease(5xFAD mice and PS19 mice) on improvement of long-term memory, two objects of the same shape and size were placed in the corner of a 42 x 42 x 25 cm box. The animal was allowed to start from the center of the box, and the time at which the animal was interested in the object was recorded for 5 minutes. After 24 hours, one of the two objects was replaced with fresh one, the times at which the mouse approached the old object and the fresh object were measured, and the preference for the new object was analyzed and used as a long-term memory analysis index.

### Statistical processing

All data were analyzed using a two-tailed T-test or ANOVA using GraphPad Prism 6 software. Post-hoc analysis was performed with Tukey's multiple comparison test, and significance was set at p<0.05. Data are indicated as mean ± S.E.M. (* p<0.05, ** p<0.01, *** p<0.001).

### <Example 1>

### Analysis of cytotoxicity of abemaciclib on BV2 microglia and reduction of pro-inflammatory cytokine levels

To determine whether or not abemaciclib is cytotoxic to BV2 microglia, BV2 microglia were treated with abemaciclib at different concentrations (0.1, 1, 5, 10, 25, or 50 µM) and then MTT assay was performed.

As can be seen from FIGS. 1A and 1B, the result of the analysis showed that abemaciclib did not induce cytotoxicity to BV2 microglia.

In addition, in order to determine whether or not abemaciclib affects the expression level of pro-inflammatory cytokines induced by LPS, BV2 microglia were pre-treated with abemaciclib (5 µM) or vehicle (1% DMSO) for 30 minutes and then treated with LPS (1 µg/ml) or PBS for 5.5 hours, and mRNA levels of pro-inflammatory cytokines were analyzed. At this time, as another experiment, BV2 microglia were pretreated with LPS (1 µg/ml) or PBS for 30 minutes, treated with abemaciclib (5 µM) or vehicle (1% DMSO) for 5.5 hours, and then the mRNA levels of the pro-inflammatory cytokines IL-6, IL-1beta, iNOS, and COX-2 were analyzed.

As can be seen from FIGS. 1C to 1G, the result shows that when inflammation was induced with LPS and then treated with abemaciclib, the production of pro-inflammatory cytokines IL-6, IL-1beta, iNOS, and COX-2 was effectively inhibited (FIGS. 1H to 1L).

Therefore, these results indicate that abemaciclib can act both before and after the induction of an inflammatory response to inhibit the production of proinflammatory cytokines. In addition, in order to determine whether or not abemaciclib also affects the anti-inflammatory response, BV2 microglia were pretreated with abemaciclib (5 µM) or vehicle (1% DMSO) for 30 minutes and then treated with LPS (1 µg/mL) or PBS for 5.5 hours, and the mRNA levels of IL-4, IL-10 and MRC-1 were measured.

Also, at this time, mRNA levels of IL-4, IL-10 and MRC-1 were measured for the group in which BV2 microglia were pre-treated with LPS (1 µg/mL) or PBS for 30 minutes and then treated with abemaciclib (5 µM) or vehicle (1% DMSO) for 5.5 hours.

As can be seen from FIGS. 1M to 1R, abemaciclib greatly increased IL-4, IL-10, and MRC-1 mRNA levels in LPS-treated BV2 microglia. Even pretreatment with abemaciclib and then treatment with LPS significantly increased the mRNA levels of anti-inflammatory factors IL-4, IL-10, and MRC-1.

Therefore, based on these results, the present inventors found that abemaciclib has effects of effectively inhibiting the expression and production of LPS-induced pro-inflammatory cytokines and promoting the expression and production of anti-inflammatory cytokines, thus being useful for the prevention, amelioration and treatment of degenerative brain diseases and brain inflammation-related diseases due to brain inflammation that may be caused by an inflammatory response.

### <Example 2>

### Analysis of effect of abemaciclib on TLR4/AKT/STAT3 signaling

In order to determine the signaling pathway though which abemaciclib can modulate the LPS-induced inflammatory response, the effect of abemaciclib on TLR4 signaling was analyzed. To this end, BV2 microglia were first cultured, starved for 1 hour, treated with TAK-242 (TLR4 inhibitor, 500 nM) or vehicle (1% DMSO) for 30 minutes, and then treated with abemaciclib (5 µM) or vehicle (1% DMSO) for 30 minutes, followed by treatment with LPS (1 µg/ml) or PBS for 5 hours. Then, the level of pro-inflammatory cytokines for each experimental group was analyzed by RT-PCR.

The result shows that the groups treated with abemaciclib significantly reduced the mRNA levels of IL-6, IL-1β, iNOS, and COX-2, which are pro-inflammatory cytokines induced by LPS compared with vehicle treatment. In particular, the group treated with a combination of TAK-242 and abemaciclib had no significant change in iNOS mRNA level compared with treatment with LPS and abemaciclib or treatment with TAK-242 and LPS(FIGS. 2A to 2E). Therefore, this means that abemaciclib inhibits iNOS mRNA level, a final product of the LPS-induced inflammatory response, depending on the TLR4 signaling pathway.

In addition, in order to determine the intracellular signaling pathway of the inhibition of LPS-mediated inflammatory response by abemaciclib, BV2 microglia were cultured, starved for 1 hour, and then treated with abemaciclib (5 µM) or vehicle (1% DMSO) for 45 minutes, followed by treatment with LPS (1 µg/ml) or PBS for 45 minutes. Then, the levels of AKT and p-AKT (phosphorylated) were analyzed by Western blotting.

The result shows that abemaciclib significantly reduced the level of p-AKT induced by LPS in BV2 microglia treated with abemaciclib, but it did not affect the total expression level of AKT protein (FIGS. 2F to 2H).

Next, to determine whether or not abemaciclib inhibits the level of LPS-mediated proinflammatory cytokines in an AKT-dependent manner, BV2 microglia were treated with MK2206 (AKT inhibitor, 10 µM) or vehicle (1% DMSO) for 30 minutes and then treated with abemaciclib (5 µM) or vehicle (1% DMSO) for 30 minutes, followed by LPS (1 µg/ml) or PBS for 5 hours.

The result of analysis of the levels of pro-inflammatory cytokines shows that the group treated with all of MK2206, abemaciclib, and LPS was not significantly different in the mRNA levels of iNOS, IL-1beta, and COX-2 induced by LPS from other groups (FIGS. 2I to 2M). These results indicate that abemaciclib inhibits LPS mediated iNOS, IL-1beta, and COX-2 mRNA level in an AKT-dependent manner to downregulates, thereby inhibiting neuroinflammation.

In addition, to determine whether or not abemaciclib can modulate p-STAT3 levels in the cytoplasm and nucleus, BV2 microglia were treated with abemaciclib (5 µM) or vehicle (1% DMSO) for 30 minutes and then treated with LPS for 5.5 hours, cell lysates were obtained from each experimental group and fractionated into cytosolic and nuclear fractions, and then the level of p-STAT3 was analyzed by Western blotting.

As can be seen from FIGS. 2N to 2Q, the result shows that abemaciclib significantly reduced p-STAT3 levels in the nucleus, whereas it had almost no change p-STAT3 levels in the cytosol. Therefore, this means that abemaciclib of the present invention inhibits nuclear p-STAT3 levels and thus can ultimately inhibit the activation of STAT3 in the nucleus. This indicates that phosphorylation of STAT3 (p-STAT3) is known to function to induce the expression of inflammatory factors in the nucleus and the reduction in the level of p-STAT3 inhibits the expression of inflammatory factors, thus ultimately suppressing excessive inflammatory responses.

### <Example 3>

### Analysis of the effect of treatment of microglia and astrocytes with abemaciclib on inhibition of LPS-induced pro-inflammatory cytokines

In order to determine whether or not abemaciclib in microglia is related to LPS-induced neuroinflammation, primary microglia were cultured under low glucose conditions, pre-treated with abemaciclib (5 µM) or vehicle (1% DMSO) for 30 minutes and treated with LPS (200 ng/ml) or PBS for 5.5 hours, and real-time PCR was performed to measure levels of pro-inflammatory cytokines.

As shown in FIG. 3A, the result shows that the levels of pro-inflammatory cytokines induced by LPS were significantly reduced in primary microglia treated with abemaciclib.

In addition, the present inventors determined whether or not abemaciclib could modulate the LPS-induced neuroinflammatory response in primary astrocytes under the same drug treatment conditions. The result shows that mRNA levels of the pro-inflammatory cytokines COX-2, IL-1β, IL-6, TNF-a and iNOS were reduced (FIG. 3B).

These results indicate that abemaciclib can effectively inhibit LPS-induced neuroinflammatory responses in both microglia and astrocytes.

### <Example 4>

### Analysis of effects of abemaciclib on LPS-induced cognitive decline in wild-type mice

In order to determine whether or not abemaciclib improves memory decline induced by repetitive inflammatory stimuli in wild-type mice, abemaciclib (30 mg/kg, intraperitoneal administration) or vehicle (10% DMSO) daily for 7 days was administered to wild-type mice, 30 minutes later, LPS (250 µg/ml, intraperitoneal administration) or PBS was administered thereto, Y maze and NOR training were performed on the 8th day, and the NOR test was performed on day 9. During the behavioral test period (8 and 9 days), abemaciclib and LPS were administered thereto after the behavioral test.

As can be seen from FIGS. 4A to 4B, treatment with abemaciclib improved LPS-mediated spatial memory (analyzed by performing the Y maze) and cognitive memory impairment (analyzed by performing the NOR test) in wild-type mice.

### <Example 5>

### Analysis of effects of abemaciclib on LPS-induced microglia/astrocyte activation and pro-inflammatory cytokine levels in wild-type mice

Whether or not abemaciclib could modulate LPS-induced neuroinflammation in vivo was determined. To this end, wild-type mice (normal mice) were injected daily with abemaciclib (30 mg/kg, ip) or vehicle (10% DMSO) for 3 days and then treated with LPS (10 mg/kg, ip) or PBS for 6 hours. Then, the cortex and hippocampus were isolated from the mice, and were subjected to immunohistochemical staining using anti-lba-1, anti-GFAP antibodies, and anti-IL-1β antibodies.

As can be seen from FIGS. 5A and 5C, the result shows that the abemaciclib-treated group exhibited significant reduction in LPS-induced Iba-1 in the cortex, whereas the reduction of Iba-1 by treatment with abemaciclib of the hippocampus was found to be weaker than that of the cortex. Therefore, these results showed that the present inventors found that abemaciclib can inhibit microglia activation.

In addition, it was determined whether or not abemaciclib could inhibit activation of astrocytes induced by LPS. As shown in FIGS. 5B to 5C, the result shows that GFAP, which is a marker of LPS-induced astrocyte activation, was significantly reduced in the cortex, whereas the reduction in GFAP in the hippocampus was less than that of the cortex.

In addition, whether or not abemaciclib affected the level of pro-inflammatory cytokines induced by LPS was determined. As can be seen from FIGS. 6A and 6C, the result showed that abemaciclib treatment significantly reduced LPS-induced IL-1β levels in the cortex and hippocampus CA1 region (hippocampus). As can be seen from FIGS. 6B and 6C, LPS-induced IL-6 was also significantly reduced in the cortex of abemaciclib treated wild-type mice.

Therefore, based on these results, the present inventors found that abemaciclib can inhibit activation of microglia and astrocytes induced by LPS in wile type mice, and inhibit the production and expression of pro-inflammatory cytokines, thereby preventing the induction of neuroinflammation in brain nerve cells.

### <Example 6>

### Analysis of effects of abemaciclib on amyloid-beta-induced microglia/astrocyte activation and pro-inflammatory cytokine levels in mice model of degenerative brain disease

As confirmed through Example 5 that abemaciclib suppress neuroinflammation in normal mice, the present inventors performed an experiment on mice induced with Alzheimer's disease, one of the degenerative brain diseases (5x FAD mice), to determine whether or not treatment with abemaciclib had inhibitory activity against neuroinflammation. To this end, after intraperitoneal administration of abemaciclib (30 mg/kg, ip) or vehicle (10% DMSO) daily for 2 weeks to 5x FAD mice, an animal model of Alzheimer's disease immunohistochemical staining was performed in the same manner as in Example 5.

As can be seen from FIGS. 7A and 7D, the result shows that the abemaciclib-treated group significantly reduced Iba-1, a marker of microglial activation in the cortex and hippocampus, and as can be seen from FIGS. 7B and 7D, the abemaciclib-treated group significantly reduced GFAP, which is a marker of astrocyte activation, compared to the control group, whereas the decrease in GFAP in the hippocampus was less than that of the cortex.

In addition, as can be seen from FIGS. 7C and 7D, the abemaciclib-treated group significantly reduced the inflammatory cytokine, IL-1β, in the cortex, compared to the vehicle-treated group.

Therefore, abemaciclib can inhibit the activation of microglia and astrocytes in the neurons of the brain where neurodegenerative diseases is induced, and suppress the production and expression of pro-inflammatory cytokines, thus being useful as a potential therapeutic agent for neurodegenerative diseases.

### <Example 7>

### Analysis of effects of abemaciclib on cognitive ability and dendritic spine density increase in brain tissues of animal model of neurodegenerative disease

To determine whether or not abemaciclib affects learning and memory and dendritic spine formation, abemaciclib (30 mg/kg, ip) or vehicle (10% DMSO) was administered to animal model of Alzheimer's disease. After intraperitoneal administration, Y maze test was performed on day 15, NOR training was performed on day 16, and NOR test was performed on day 17. In addition, the number of dendritic spines in the cortical and hippocampal neurons of the mice was measured.

As can be seen from FIGS. 8A and 8B, the result shows that treatment with abemaciclib improved short-term memory and long-term memory in animal model of neurodegenerative disease and, as can be seen from FIGS. 8C to 8E, abemaciclib-treated group exhibited the increased number of dendritic spines in the cerebral cortical and hippocampal AO regions compared to the control group. These results were also confirmed from the Golgi staining image as well.

Therefore, abemaciclib of the present invention can increase the number of dendritic spine in a mouse model of neurodegenerative disease and improved cognition and memory deteriorated by degenerative brain disease, thus being useful as a potential therapeutic agent for neurodegenerative disease.

### <Example 8>

### Analysis of effect of abemaciclib on inhibitory activity of tau phosphorylation

In order to determine the effect of abemaciclib on tau phosphorylation, abemaciclib (30 mg/kg, ip) or vehicle (10% DMSO) was administered to Alzheimer's disease-induced 3-month-old 5xFAD mice daily for 2 weeks. Then, immunohistochemistry analysis was performed using anti-AT100 antibody, anti-AT180 antibody, and anti-Tau-5 antibody that can be used to detect phosphorylated tau protein.

The result shows that abemaciclib-treated 5xFAD mice significantly reduced phosphorylated tau protein detected by AT100-antibody in the cerebral cortex, compared to the vehicle-administered control group. In addition, abemaciclib-treated 5xFAD mice significantly reduced tau phosphorylation detected by anti-AT180 and anti-Tau-5 antibodies in the cerebral cortex and hippocampus, compared to the vehicle-administered control group(FIGS. 9A to 9D).

Therefore, these results indicate that abemaciclib can prevent or treat degenerative brain diseases such as Alzheimer's disease by effectively inhibiting tau phosphorylation known as the cause of neurodegenerative diseases.

### <Example 9>

### Analysis of effect of abemaciclib on Aβ plaque reduction in mice with neurodegenerative disease

In order to determine whether or not abemaciclib affects the pathogenesis of amyloid plaques (Aβ), abemaciclib (30 mg/kg, ip) or vehicle (10% DMSO) was injected into 3-month-old 5x FAD mice daily for 2 weeks, and then cortical and hippocampal tissues were isolated from the mice, respectively, and changes in the level of amyloid plaques (Aβ) were measured.

As can be seen from FIG. 10, the result shows that abemaciclib-treated Alzheimer's disease mouse model significantly reduced the level of amyloid plaques (Aβ) in the CA1 region compared to the vehicle-treated control group.

### <Example 10>

### Comparison between abemaciclib and donepezil in effect of improving cognitive function in mice model of neurodegenerative disease

In order to compare the effect of abemaciclib in controlling the symptoms of neurodegenerative disease with donepezil, abemaciclib (30 mg/kg, intraperitoneal administration), donepezil (1 mg/kg, intraperitoneal administration) or vehicle (10% DMSO), a currently marketed therapeutic agent for dementia, was administered to the 5xFAD mice daily for 14 days and then Y maze test on day 15 and NOR test on day 16-17 were performed.

As can be seen from FIGS. 11A and 11B, the group administered donepezil improved short-term memory and long-term memory in mice model of Alzheimer's disease, and the treatment with abemaciclib mesylate also improved short-term and long-term memory in mice model of neurodegenerative disease, comparable to donepezil.

### <Example 11>

### Comparison between abemaciclib and donepezil in the astrocyte activity inhibitory effect in mice model of neurodegenerative disease

In order to compare the effect of abemaciclib in controlling the symptoms of neurodegenerative disease with donepezil, abemaciclib (30 mg/kg, intraperitoneal administration), donepezil (1 mg/kg, intraperitoneal administration) or vehicle (10% DMSO), a currently marketed therapeutic agent for dementia, were administered to the 5xFAD mice daily for 14 days, behavioral experiments were performed on days 15 to 17 and immunohistochemical analysis was performed.

As can be seen from FIGS. 12A and 12B, administration with donepezil reduced astrocyte activation and proliferation of astrocytes in the cerebral cortex of mice with neurodegenerative disease, and also reduced the number of astrocytes directly interacting with amyloid plaques. Also, the group administered abemaciclib inhibited activity and proliferation of astrocytes and reduced the number of astrocytes interacting with amyloid plaques to a level similar to that of the donepezil-administered group.

### <Example 12>

### Analysis of effect of abemaciclib on inhibition of tau kinase activity in mice model of neurodegenerative disease compared to donepezil

In order to determine the effect of abemaciclib on control of the symptoms of neurodegenerative disease with donepezil, a currently marketed therapeutic agent for dementia, abemaciclib (30 mg/kg, intraperitoneal administration), donepezil (1 mg/kg, intraperitoneal administration) or vehicle (10% DMSO) were administered to 5xFAD mice daily for 14 days, behavioral experiments were performed on days 15 to 17, and immunohistochemical analysis was performed.

As can be seen from FIG. 13A, the result shows that administration of donepezil reduced hippocampal phospho-GSK-3β levels in mice model of neurodegenerative disease, and administration of abemaciclib reduced hippocampal phospho-GSK-3β levels compared to administration of donepezil. Moreover, as shown in FIG. 13B, administration of donepezil reduced the expression of DYRK1A, a tau kinase, in the cortex and hippocampus of mice model of neurodegenerative disease, and administration of abemaciclib was highly effective in inhibiting the expression of DYRK1A, a tau kinase, in the cortex and hippocampus, compared to administration of donepezil.

### <Example 13>

### Confirmation of effects of abemaciclib on cognitive function improvement in tau-overexpressing mice model of neurodegenerative disease

Since abemaciclib had effects of reducing tau phosphorylation and inhibiting tau kinase activity in the 5xFAD mice, an animal model of neurodegenerative disease overexpressing APP, in order to determine whether abemaciclib regulates cognitive function in PS19 mouse, an animal model of tau-overexpressing neurodegenerative disease was determined. For this purpose, abemaciclib (30 mg/kg for intraperitoneal administration or oral administration for 60 mg/kg) or vehicle (10% DMSO) was administered to 6-month-old PS19 mice daily for 14 days, and then NOR test was performed on day 15 and the Y maze test was performed on the days 15 and 16.

As can be seen from FIGS. 14A and 14B, the result shows that administration of abemaciclib improved spatial perception ability and recognition memory function of mice induced with degenerative brain disease due to overexpression of tau.

Based on the above results, the present inventors found that abemaciclib is capable of effectively inhibiting the expression and production of LPS-induced pro-inflammatory cytokines, inhibiting the LPS-induced activation of microglia and astrocytes, and effects of inhibiting plaque formation, increasing the number of dendritic spines, improving long-term memory, and inhibiting phosphorylation of tau protein, thus being useful as a novel therapeutic agent for preventing, ameliorating, or treating degenerative brain diseases.

The present invention has been described with respect to preferred embodiments thereof. Those skill in the art to which the present invention pertains will appropriate that the present invention can be implemented in a modified form without departing from the essential characteristics of the present invention. Therefore, the disclosed embodiments should be considered from an explanatory point of view rather than a limiting point of view. The scope of the present invention falls within the claims rather than the foregoing description and all differences within the equivalent scope will be construed as being incorporated in the present invention.

## Claims

1. A pharmaceutical composition for preventing or treating a degenerative brain disease comprising abemaciclib or a pharmaceutically acceptable salt thereof as an active ingredient.

2. The pharmaceutical composition according to claim 1, wherein the composition inhibits expression and production of inflammatory cytokine including COX-2, IL-1β, IL-6 or iNOS.

3. The pharmaceutical composition according to claim 1, wherein the composition inhibits activity of toll-like receptor 4 (TLR4) and reduces a level of p-STAT3.

4. The pharmaceutical composition according to claim 1, wherein the composition inhibits activity of microglia or astrocytes, inhibits amyloid plaques, increases the number of dendritic spines, inhibits phosphorylation of tau protein, or improves long-term memory.

5. The pharmaceutical composition according to claim 1, wherein the degenerative brain disease is selected from the group consisting of Alzheimer's disease, Parkinson's disease, Huntington's disease, multiple sclerosis, multiple system atrophy, epilepsy, encephalopathy, cognitive impairment, learning disability, memory impairment, and stroke.

6. A health functional food for preventing or ameliorating a degenerative brain disease comprising abemaciclib or a salt thereof as an active ingredient.

7. The health functional food according to claim 6, wherein the abemaciclib or salt thereof inhibits expression and production of inflammatory cytokines including COX-2, IL-1β, IL-6 or iNOS.

8. The health functional food according to claim 6, wherein the abemaciclib or salt thereof inhibits activity of toll-like receptor 4 (TLR4) and reduces a level of p-STAT3.

9. The health functional food according to claim 6, wherein the abemaciclib or salt thereof inhibits activity of microglia or astrocytes, inhibits amyloid plaques, increases the number of dendritic spines, inhibits phosphorylation of tau protein, or improves long-term memory.

10. The health functional food according to claim 6, wherein the degenerative brain disease is selected from the group consisting of Alzheimer's disease, Parkinson's disease, Huntington's disease, multiple sclerosis, multiple system atrophy, epilepsy, encephalopathy, cognitive impairment, learning disability, memory impairment, and stroke.

11. A pharmaceutical composition for preventing or treating cognitive impairment, learning disability or memory impairment comprising abemaciclib or a pharmaceutically acceptable salt thereof as an active ingredient.

12. A health functional food for preventing or ameliorating cognitive impairment, learning disability or memory impairment comprising abemaciclib or a salt thereof as an active ingredient.

13. A pharmaceutical composition for preventing or treating a neuroinflammatory disease comprising abemaciclib or a pharmaceutically acceptable salt thereof as an active ingredient.

14. The pharmaceutical composition according to claim 13, wherein the neuroinflammatory disease is selected from the group consisting of neuroblastoma, Lou Geh-rig's disease, Creutzfeldt-Jakob disease, post-traumatic stress disorder, depression, schizophrenia, and amyotrophic lateral sclerosis.

15. A health functional food for preventing or ameliorating a neuroinflammatory disease comprising abemaciclib or a pharmaceutically acceptable salt thereof as an active ingredient.

16. The health functional food according to claim 15, wherein the neuroinflammatory disease is selected from the group consisting of neuroblastoma, Lou Gehrig's disease, Creutzfeldt-Jakob disease, post-traumatic stress disorder, depression, schizophrenia, and amyotrophic lateral sclerosis.
